# EUROPEAN PATENT APPLICATION

(11) **EP 2 316 513 A2**
(43) Date of publication of application: **04.05.2011**
(21) Application number: 10188012.8
(22) Date of filing: 19.10.2010
(51) Int. Cl.: A61M 5/32, A61M 5/00

(54) **Kits comprising syringe assemblies**

(30) Priority: 19.10.2009 US 252962 P
(71) Applicant: Terumo Medical Corporation, Somerset, New Jersey 08873 (US)
(72) Inventor: Powers, Thomas, Newtown Pennsylvania 18940 (US); Dowdell, Ralph, Trenton New Jersey 08611 (US); Hidalgo, Craig, Langhorne Pennsylvania 19047 (US); Bredael, Gary, Langhorne Pennsylvania 19053 (US); Mathews, David, Joppa Massachusetts 21085 (US); Voellmicke, Craig, New York New York 10023 (US)
(74) Representative: Jenkins, Peter David

(57) **Abstract**

The present invention is directed to kits comprising syringe assemblies having detachable needle assemblies, as well as shield apparatuses to protect the needle assemblies. The syringe assemblies are configured to minimize the dead space after deployment of the syringe and injection of the material in the syringe into a patient's body, as well as to include detachable needle assemblies, such that different needle assemblies can be interchanged on a single syringe barrel.

## Description

### CROSS REFERENCE TO RELATED APPLICATIONS

This application claims benefit of U.S. Provisional Application No. 61/252,962 filed October 19, 2009, hereby incorporated by reference in its entirety.

### BACKGROUND OF THE INVENTION

The present invention is related to the field of hypodermic syringes and needles, and particularly to syringe assemblies having detachable needles, low dead space and a bevel indicator; as well as shield apparatuses for protecting and storing such syringe assemblies; as well as kits comprising syringes and interchangeable needle assemblies; all of which increase the ease and convenience of use for medical staff and patients that use such syringes to provide multiple injections over a short period of time.

Hypodermic syringes are commonly used by doctors, nurses and other medical staff (including staff trained in cosmetic procedures) for various procedures that require injection of medication and other medical or cosmetic substances into patients' bodies. Such syringes are also commonly used by certain patients, as for example, diabetics who may self-inject insulin.

In certain situations where a patient must be given multiple injections, for example for allergy injections, injections of insulin for diabetics or cosmetic applications, it is desirable to avoid dulling of a hypodermic needle over multiple uses, as dulling can lead to decreased accuracy in drug delivery and patient discomfort. Further, in view of recent industry-wide attention to minimizing the environmental impact of medical waste, it is desirable to provide syringe assemblies that permit convenient use and interchangeability of needles with syringes, such as providing a single syringe that can be used multiple times on a single patient, where a used needle attached to the barrel of a syringe can quickly and easily be replaced with a fresh and sterile needle.

In general, an injection is administered in a manner including a "loading" step - that is, a step wherein the administrator or patient obtains a fully assembled but empty syringe assembly with the plunger fully depressed into the syringe barrel, then "loads" the syringe assembly by inserting the needle portion into a source of fluid (for example, a container that holds a reservoir of the fluid to be injected into the patient). This step is followed by the administrator or patient's pulling out the plunger by sliding it along the longitudinal axis of the barrel in a proximal direction, engaging a vacuum force to load the syringe with a predetermined amount of fluid. The needle is then withdrawn from the container, fully loaded and ready to inject into the patient. Repeated loading can also dull the needle.

Small needles have not traditionally been commonly used, but are gaining in popularity. For example, needles as small as 29-30 gauge are often used to inject insulin, and even needles as small as 31-33 gauge are now becoming more widely used (the "gauge" of a needle is a way of denoting the outer diameter of the needle, and a higher gauge indicates a small needle). Some uses for smaller gauge needles include injecting insulin, administering allergy injections and for cosmetic applications such as the administration of Botox®. Further, allergy injections and Botox® injections generally require multiple injections to a patient in a single treatment. Thus, it may be desirable to use smaller needles for comfort and convenience for the patient. However, such needles are generally fragile because of their smaller size, and tend to be undesirable for loading, as their small circumference leads to slow loading and an increased risk of breakage during the handling of the syringe before injection. Moreover, multiple injections and the need to insert the needle through a barrier the top of a bottle or vial of sterile fluid as part of the loading step may all lead to faster dulling of the needle, causing further discomfort to the patient. Thus, the needles that are desirable for certain injections because of their small size are at the same time not so desirable for loading such syringes before injection into the patient. Yet, in the syringe and needle assemblies in the current art, users generally do not have the option of using one needle to load the syringe and another to inject the fluid into the patient. While a large needle may be fast to load, it may cause greater physical pain to the patient during injection, and such pain is multiplied with multiple injections. On the other hand, a small needle can be difficult to load. Thus, a user is often left with two undesirable alternatives.

In the situations where repeated loadings and injections on a single patient are desirable, there are other disadvantages. Because many small syringe assemblies do not have detachable needles, a user may choose to use a fresh syringe for each injection to avoid dulling the needle. Discarding each syringe can be expensive and wasteful. Further, repeated injections over time may incrementally increase wasted fluid, as each injection will result in some fluid that is caught in the "dead space" in the syringe assembly rather than injected into the patient. This can be very expensive in the case of high-cost cosmetic fluid preparations such as Botox®.

Still further, many hypodermic needles are designed with a bevel tip that is cut at an angle, to facilitate smooth injection. The angle of such tip is known as the "bevel angle." Most bevel tips are cut as a "regular" bevel, which makes the tip appropriate for intramuscular delivery. However, other types of bevels include "short" bevels and "intradermal" bevels (all are based on the angle of the tip). To minimize a patient's discomfort, it is generally desirable to orient a needle so that the sharpest and farthest extending point (the "needle point"), rather than the part of the point that is closest to the hub (the "needle heel") breaks the patient's skin first. In the case of larger (lower gauge) needles known in the art, the bevel angle is visible to the user's naked eye, and therefore this is fairly easy to do. However, in the case of extremely small needles (higher gauges of higher than 25), such as those used for multiple injections as discussed herein, the bevel tip may be very small and difficult or impossible to see.

Thus, a need exists for improved syringes that have detachable and interchangeable needles, such that a used needle may be easily removed from a syringe and replaced with a fresh needle; and that also minimize dead space after injection to preserve as much fluid as possible; as well as kits comprising such syringes and needles that permit easy detachment and interchanging of such parts for users of multiple needles; as well as shield apparatuses for protecting such syringe assemblies before and after use.

### SUMMARY OF THE INVENTION

In certain embodiments, the present invention is directed to a kit comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof; and a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space; and
(b) a separate needle assembly that is capable of being detachably fixed to the distal end of the cylindrical barrel to form a syringe assembly, the needle assembly comprising a hub and a needle fixed on the hub, wherein the needle comprises a hollow lumen therethrough, the hollow lumen communicating with the interior space of the cylindrical barrel.

In other embodiments, the present invention is directed to a kit comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof; and a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space;
(b) a first needle assembly detachably fixed to the distal end of the cylindrical barrel, the needle assembly comprising a hub and a needle fixed on the hub, wherein the needle comprises a hollow lumen therethrough, the hollow lumen communicating with the interior space of the cylindrical barrel; and
(c) a shield apparatus comprising a housing having an exterior wall and an interior wall defining an interior chamber, the shield apparatus holding a second needle assembly in its interior chamber.

In other embodiments, the present invention is directed to a method of forming a syringe assembly, the method comprising the steps of:
(a) obtaining a cylindrical barrel having a proximal end and a distal end, the cylindrical barrel defining an interior space, the interior space including a distal end and a roof on the distal end, the interior space configured to receive a plunger capable of sliding relative to the cylindrical barrel;
(b) obtaining a syringe assembly, the syringe assembly comprising a hub fixed to a needle, the needle having a needle point; and
(c) detachably connecting the distal end of the cylindrical barrel onto the syringe assembly; wherein the detachable connection is achieved with a mechanism chosen from: a threading connection, a snap connection and a binary connection.

### BRIEF DESCRIPTION OF THE DRAWINGS

**Fig. 1** shows some components of a kit in accordance with one embodiment of the present invention.

**Fig. 2** shows some components of a kit in accordance with another embodiment of the present invention.

**Fig. 3** shows some components of a kit in accordance with another embodiment of the present invention.

**Figs. 4a** and **4b** show some components of a kit in accordance with another embodiment of the present invention.

### DETAILED DESCRIPTION OF THE INVENTION

The kits of the present invention have been found to be particularly useful for applications directed to unique types of syringe assemblies - specifically, syringe assemblies that exhibit the characteristics of low dead space, detachability of the needle portion, and in certain embodiments, a bevel indicator to assist a user in guiding the needle into the patient's body at the optimal point. All of these are attributes of certain embodiments of the syringe assemblies that can be held within the shield apparatuses of the present invention, and all will be discussed below. Desirable syringe assemblies are discussed in great detail in copending U.S. Application No. ________, filed on the same date as the present application and entitled "Syringe Assemblies Having Detachable Needle Assemblies and Low Dead Space" the disclosure of which is hereby incorporated by reference in its entirety. Further, shield apparatuses that hold such syringe assemblies and needle assemblies are discussed in great detail in copending U.S. Application No. _________, filed on the same date as the present application and entitled, "Shield Apparatuses and Methods for Storing Syringe Assemblies and Needle Assemblies" the disclosure of which is hereby incorporated by reference in its entirety.

Such syringe assemblies are particularly useful for the injection of medical or cosmetic fluid to treat conditions such as, but not limited to, allergies, hypohydrosis, muscle twitches, crossed eyes, cerebral palsy or the like, that require multiple injections along multiple sites on a patient's face or body in a single procedure or series of procedures. As used herein, the terms "medical or cosmetic fluid" refer to any fluid material that is desired to be delivered to a patient's body by injection using a syringe assembly, for treating a medical condition or providing a cosmetic benefit. In certain embodiments, the medical or cosmetic fluid may comprise, but is not limited to, allergenic and allergy-triggering compositions such as dander, or cosmetic compositions such as botulism toxin (in the case of cosmetic fluids such as Botox®) and muscle relaxants and the like.

### Low Dead Space

The issue of dead space is an ongoing concern in the field of syringes and other surgical instruments that deliver fluids. A primary concern is that whenever currently used syringes are fully deployed, liquid remains in the syringe, generally in the distal end of the barrel that may comprise a conical or cross-sectionally trapezoidal shape, as well as in the length of the needle itself. This liquid is lost after deployment of the syringe assembly, as there is no way to extract it from the syringe or force it from the barrel through the needle.

Attempts have been made to both minimize dead space and provide detachable needle assemblies. See, for example, U.S. Patent No. 5,782,803 to Jentzen and U.S. Patent Publication No. 2008/0033347 to D'Arrigo. However, these and other references in the art address syringes more traditionally used that have larger needles (generally gauges of far lower than 29) with a capacity of 3 mL and larger. The interchangeability and dead space of higher gauge needles has not been adequately addressed by prior attempts. As discussed herein, small gauge needles (for example, 29-33 gauge needles and even smaller) present unique challenges in the art, including but not limited to their fragility and difficulty to detach due to their small size.

It has been discovered that minimization of dead space can be achieved via the design of the plunger and syringe barrel configurations. Specifically, the interior of the barrel is configured to receive a plunger, and the plunger has a distal end (which is inserted into the barrel) and a proximal end, which in certain embodiments, comprises a plunger handle to facilitate the user's sliding the plunger along the longitudinal axis of the barrel. The cylindrical barrel comprises an interior cavity, and at the distal portion of the cavity is an interior distal surface, which the plunger touches when fully deployed - that is, fully slid into the barrel such that it can slide no farther in. This interior distal surface of the barrel that the distal end of the plunger ultimately touches when fully deployed, and which represents the farthest surface to which the plunger can travel into the barrel, is referred to herein as the "roof" of the barrel.

While at least a portion of the roof of the barrel is a closed surface in order to receive the plunger, the roof of the barrel will, in certain embodiments, comprise at least one opening that permits fluid communication of the interior of the barrel with the needle assembly, thus permitting injection of the fluid from the barrel into the body of a patient via the hollow lumen of the needle when the user depresses the plunger toward the roof. The greater the surface area of contact between the distal end of the plunger (when fully deployed) and the roof of the barrel, the less dead space results. In certain embodiments of the present invention, the distal end of the plunger and the roof both have shapes that correspond to and complement each other - that is, the distal end of the plunger has a shape that corresponds to an opposite and complementary shape on the roof of the distal portion of the interior space, such that when the plunger is fully deployed within the cylindrical barrel, the distal end of the plunger and the roof of the distal portion of the interior space mate at an interface to form a seal that forces substantially all of the fluid away from the interface and through the opening through which the interior of the barrel communicates with the needle assembly. The tighter this seal, the more dead space can be minimized and the more fluid can be forced out of the syringe barrel. This corresponding relationship between the distal end of the plunger and the roof is important to ensure that when the plunger is fully deployed into the cylindrical barrel, the plunger and the roof are in substantially full contact with each other.

In certain embodiments, the distal end of the plunger may be one of any other shapes that minimize dead space - for example, partially or substantially conical, trapezoidal, circular, angular, or any shape that is useful for coaxing the fluid down through the barrel and into the needle assembly. For example, the distal end of the plunger may be substantially flat and at a substantially perpendicular angle to the longitudinal axis of the syringe barrel; the corresponding roof of the barrel may also be substantially flat in the same way, such that when the two meet, an optimal amount of fluid may be forced out of the barrel and through the needle.

In certain embodiments, the kits of the present invention comprise a syringe assembly configured such that less than about 5% of the fluid originally loaded into the barrel is lost to dead space when the plunger of the syringe assembly is fully deployed. In various embodiments, this value is less than about 4%, less than about 3%, less than about 2%, or less than about 1%. In certain embodiments, the amount of fluid lost is less than about 0.3 mL, less than about 0.2 mL, less than about 0.1 mL or less than about 0.05 mL.

### Detachable Needle Assembly

In various embodiments, the kits of the present invention include a syringe assembly that comprises a detachable needle assembly. As used herein, "detachable" means that a user can both attach the needle assembly to, and remove the needle assembly from, the distal end of the barrel without damaging either the needle assembly or the barrel or the connection between them.

Thus, in certain embodiments, the syringe assemblies in the kits of the present invention comprise two major components: first, a cylindrical barrel having disposed therein a plunger (herein also referred to as the "barrel," "cylindrical barrel" or "syringe barrel"); and second, a needle assembly comprising a hub fixed to a needle. In certain embodiments, the needle assembly is detachably fixed to the cylindrical barrel via any of the following mechanisms:
(a) a threaded portion on the distal end of the barrel that mates with a threaded portion of the syringe assembly ("Threading Connection");
(b) a portion on the distal end of the barrel that fits with a corresponding and complementary portion on the syringe assembly by a snapping together ("Snap Connection"); or
(c) male and female portions that are capable of being engaged together and connected by twisting ("Binary Connection").

In addition to being set forth in detail in copending U.S. Application No. ,filed on the same date as the present application and entitled "Syringe Assemblies Having Detachable Needle Assemblies and Low Dead Space," these mechanisms will be further discussed in greater detail herein.

A. Threading Connection

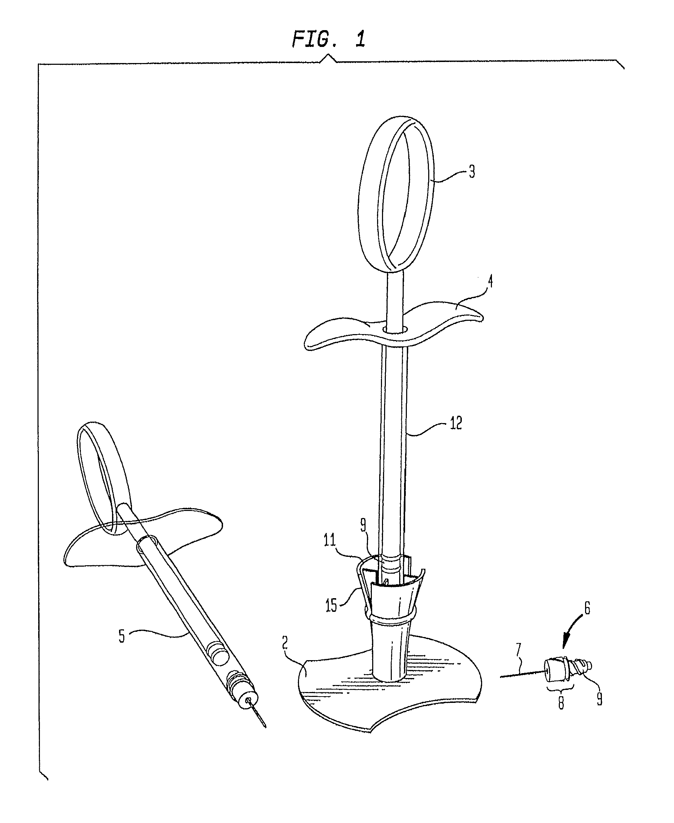

In certain embodiments, the connection between the syringe and the needle in the kits of the present invention may be made by one or more threads on the needle assembly, where the threads correspond to and mate with corresponding threads on the distal end of the syringe assembly at the point of attachment, as with a screw. Examples of a threading connection can be seen, *e.g*., in **Fig**. **1**. In **Fig. 1**, the threads 9 can be seen in three places: disposed within the barrel in the fully assembled syringe apparatus that is held within a shield apparatus 1 in the center, in the separate syringe assembly 6 on the right, and in the separate syringe apparatus on the left. The needle assembly 6 comprises the hub 8 fixed to the needle 7. When the needle assembly and the distal end of the syringe assembly are fully threaded together, the needle 7 is in seamless fluid communication with the interior of the barrel 12. In certain embodiments, the threading mechanisms connecting the needles to the syringes may further comprise one or more attachments that serve the purpose of locking the two together after threading, such that movement or pushing of the syringe or needle does not cause the needle to "walk off the syringe - that is, that inadvertent unthreading does not occur. Such a locking mechanism may be in the form of a snap, a hook, a clamp flange or other similar mechanism, as well as adhesive or a magnetic attachment.

B. "Snap" Connection

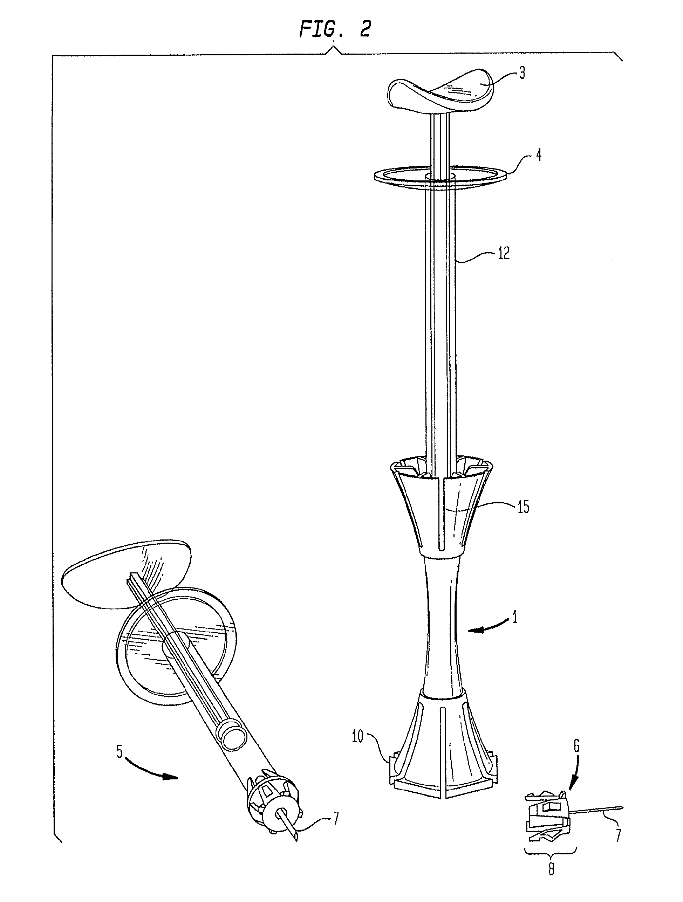

In certain embodiments, the connection between the syringe assembly's barrel and the needle assembly may be made with corresponding mating parts on the needle (or attachment thereto) and the syringe, for example, "male" and "female" parts that mate together by a "click" or "snap" mechanism. In such embodiments, either of the needle assembly or the distal end of the barrel may be configured to have a slightly larger diameter than the other, such that one of the two can fit over the other. In certain embodiments, both may be of substantially the same diameter, but either of the needle assembly or the distal end of the barrel may be configured to deform and then pop into place in a manner that the two are not easily separated during use of the syringe assembly and insertion of the needle assembly portion into the shield apparatuses of the present invention; in certain embodiments, may be locked or joined tightly into place. An example of a snap connection can be seen in **Fig. 2****.** The hub 8 includes snap enclosures that engage complementary and corresponding portions on the distal end of the syringe barrel to lock together.

C. Binary Connection

In various embodiments, the connection is of a binary nature - that is, the corresponding ends of the syringe or needle (or attachment thereto) have locking portions (for example, a flange or a lip) that can be mated by inserting one portion into a corresponding and complementary portion of another and then making a "twist" to lock the two in place. Generally, the "twist" action may be less than a full 360 degree twist - that is, a fraction of the outer circumference of the locking portions. In various embodiments, the "twist" action can be accomplished by twisting to an amount of about 45 to about 270 degrees, about 50 to about 180 degrees, about 55 to about 120 degrees or about 45, about 90, about 120, about 180 or about 270 degrees. By "binary" it is meant that the corresponding parts can be configured in two possible ways - locked and unlocked or fixed and unfixed or attached and unattached.

### Shield Apparatus

In certain embodiments of the present invention, the kits comprise a shield apparatus. The shield apparatus comprises a housing having an exterior wall and an interior wall defining an interior chamber that encloses the needle point. In certain embodiments, the shield apparatus is capable of freely standing on a horizontal plane, such that when the shield apparatus stores a syringe assembly, the needle point is facing substantially vertically downward.

In accordance with certain embodiments of the present invention, a user can easily detach and interchange needles by inserting the end of a used needle into a chamber, giving the syringe a fractional turn (in various embodiments, a quarter turn, a half turn or a turn of about 45 degrees, about 90 degrees, about 120, about 180 or about 270 degrees), thereby detaching the used needle. The user can then leave the needle assembly portion held within the chamber and insert the remainder of the syringe assembly into a different chamber, give a similar turn and engage a fresh needle that is ready for use once it is pulled out of the chamber.

### Bevel Indicator

As discussed herein, most needles comprise a beveled tip, such that the tip is at an angle. Bevel tips are highly desirable, and in certain embodiments, it is desirable to inject the needle with its bevel point entering the patient first, to minimize discomfort. While for larger needles (for example, needles having 18-25 gauge) the user can make this determination with the naked eye, this is almost impossible to do with small needles. Thus, in certain embodiments of the present invention, the needle hub, or any other point along the needle or syringe assembly, may comprise a bevel indicator. In particular, where the syringe assembly is detachable from the barrel, in certain embodiments the bevel indicator is present at least in part on the hub, and may be a line or a dot or other easily discernible indicator, and may be colored in a manner that its contrast with the color of the hub, and/or raised or recessed or having a texture in a manner that its contrast with the texture of the hub and thus visible or otherwise discernible to the user. In this manner, the user can use the bevel indicator to determine the best direction from which to inject the needle into the patient's body.

Similarly, in certain embodiments, the kits of the present invention may comprise syringe assemblies, needle assemblies or shield apparatuses, any of which may exhibit markings (for example, lines, dots, raised areas, textured areas) or other indicia that indicate to a user, among other things: the size of the needle assembly held therein, the status of the needle assembly (whether "fresh" or used); the direction or configuration of the needle point; or identifying information signifying the size (gauge) or type of needle. For example, as can be seen in **Fig. 1**, **Fig. 2** and **Fig. 3**, a slit 15 is present on a portion of the longitudinal axis of the shield apparatus in that embodiment. These slits make it possible for a user to see the interior of the shield apparatus, and may be useful in displaying the information described in this paragraph.

As a further example, each chamber may contain a mechanical trigger, such that upon insertion of a used needle, a color-coded panel or other indicator (*e.g*., a word, picture or shape) is displayed on the shield apparatus, triggered by mechanical action. This can serve the purpose of notifying the user that the needle contained therein has been used and should not be touched again. It can also indicate to a user, in the case of a multiple-chamber shield apparatus, when it is time to discard the shield apparatus and obtain a new one.

### Using the Kits of the Present Invention

In various embodiments, the present invention is directed to kits comprising one or more syringe assemblies including detachable needle assemblies, in any size or number. For example, a kit may comprise any of the following:

1. A complete syringe assembly fully loaded with a desired amount of fluid and ready to be injected into the body of a patient, along with a shield apparatus that includes one or more chambers, at least one of which is configured to receive the needle assembly that is already attached to the syringe assembly. The syringe assembly may be separate from the shield apparatus, or it may be held within the shield apparatus (as shown, for example, in the middle configurations in **Fig. 1**, **Fig. 2** and **Fig. 3**. In this case, the user can administer the injection to the patient, then insert the syringe assembly into an empty chamber of the shield apparatus, and detach the needle assembly from the barrel, leaving the used needle assembly in the chamber. In embodiments where there is more than one chamber and at least one additional fresh needle assembly, the user can then attach that fresh needle assembly to the barrel and administer a second injection. When the final needle assembly has been used, the user can safely dispose of the needle assembly be reinserting it into its chamber, detaching it from the barrel and then disposing of all parts. Alternatively, the user may leave the barrel attached to the final needle assembly when the needle assembly is held within the chamber, and discard it as one unit.

2. A syringe barrel separated from any needle assembly, along with a shield apparatus in the kit, the shield apparatus comprising one or more chambers having one or more separate needle assemblies disposed therein, and optionally with a container of fluid (for example, a bottle). In this embodiment, the user may first take the syringe barrel and attach it to a first needle assembly, and then load the fluid into the completed syringe assembly. In certain embodiments, the first needle assembly will include a lower gauge (larger) needle, and after the loading step the user may choose to insert the syringe assembly into an empty chamber of the shield apparatus, and detach the first needle assembly from the barrel, leaving the used needle assembly in the chamber; after which he may insert the barrel into a second chamber holding a second needle assembly including a higher gauge (smaller) needle, attach the second needle assembly and use it to inject the fluid into the patient.

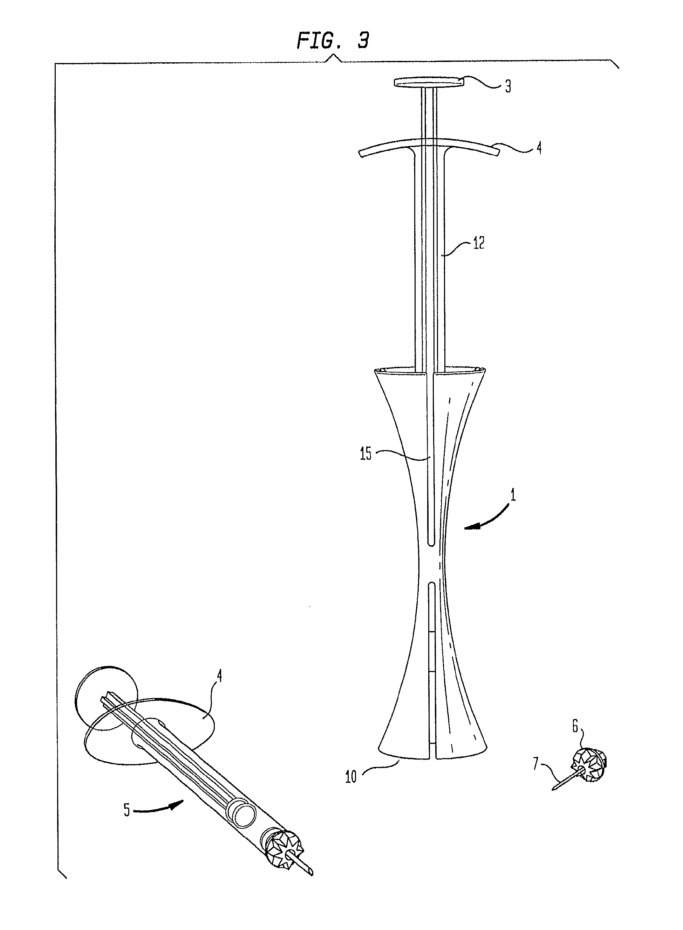

In the kits of the present invention, the needles in each needle assembly may be of any size commonly used in the art - for example, smaller gauge (larger) needles (*e.g*., 25 gauge or lower, 18 to 25 gauge, or 18, 20 or 22 gauge; or higher gauge (smaller) needles; (*e.g*., higher than 25 gauge, 26-34 gauge, or 29, 30, 31, 32 or 33 gauge). Moreover, the one or more chambers can be disposed in any of a number of configurations within the shield apparatuses. For example, a kit of the present invention may include a shield apparatus with a single chamber and a substantially flat face 2 that enables a user to stand the syringe apparatus up on its end. Alternatively, a kit of the present invention may include a shield apparatus having two separate chambers disposed on opposite ends of the shield apparatus, as shown in **Fig. 2** and **Fig. 3**, where there is no substantially flat face, but the bottom portion comprises a substantially flat plane 10 formed by the edges of engaging fins 11.

As shown in **Fig. 1** to **Fig. 3**, a kit in accordance with the present invention may include a syringe assembly already disposed within a shield apparatus 1. This syringe assembly can, in various embodiments, be already loaded with fluid and ready to inject into the patient, or can be empty, in which case in some embodiments, the kit may further include a container filled with the fluid desired to be injected. In certain embodiments, two syringe assemblies may be inserted, one in a chamber on each end of the shield apparatus. In other embodiments, any number of chambers may be disposed within the shield apparatus; for example, clustered on one end of the shield apparatus, disposed around the outer perimeter of a substantially spherical shield apparatus, or disposed in rows or columns on the edge of a shield apparatus.

Another exemplary embodiment of a kit of the present invention is seen in **Fig**. **4a**. There, it can be seen that a tray 16 is presented, having numerous recesses 17 and disposal slots 18. The tray may be made, in certain embodiments, of material such as an injection molded material or metal. The recesses 17 and disposal slots 18 may be, in certain embodiments, formed by individual shield apparatuses disposed within the tray 16; in other embodiments they may be directly built into the base instead of separate free standing apparatuses. An advantage of such a kit is that once a syringe assembly has been used, it can be inserted into the empty chamber and released therein, such that the user never has to touch the needle portion of the needle assembly to detach or attach it. When all of the syringe assemblies have been used, the tray 16 may be recycled or discarded.

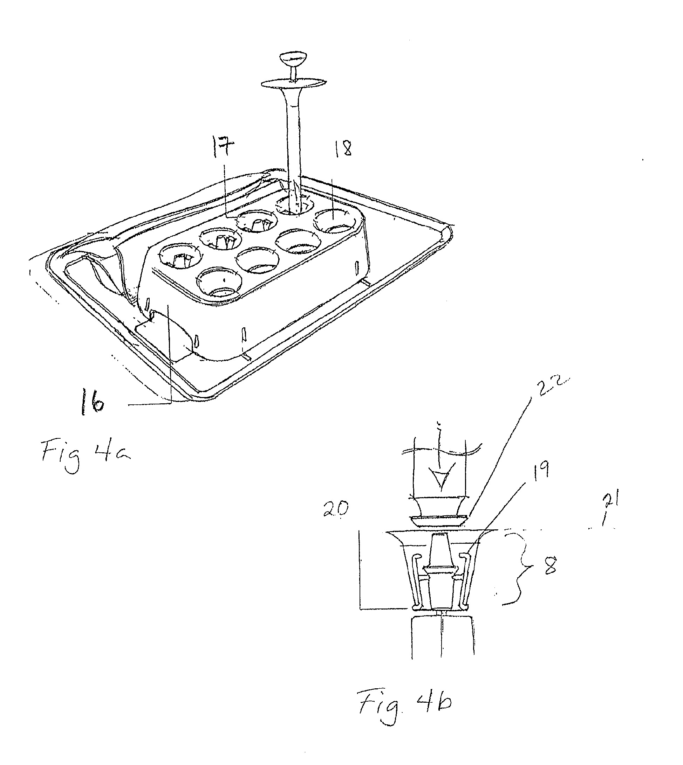

In the particular embodiment depicted in **Fig. 4a**, an exemplary syringe assembly is shown that may be useful in the kits of the present invention. **Fig. 4a** shows the internal cross sectional view of such needle assembly. As can be seen, the interior of the hub comprises one or more toggles 19. Each toggle has one or more toggle lips 20, which catch the needle within the hub and hold it in place. When stored in the tray 16, the proximal portion of the hub 8 is substantially in alignment with the top 21 of a recess (as shown by the dotted line). The user can easily insert the syringe barrel into the opening and lock the needle assembly onto the barrel (as shown by the downward arrow in **Fig. 4b**). In this embodiment, the hub comprises a barrel snap ring 22, which catches under one or more of the toggles 19, holding the syringe barrel in place. Removal can be accomplished with equal convenience.

In other embodiments, the kits of the present invention comprise a shield apparatus that comprises two or more chambers, each including a fresh detachable needle that may be different from, or identical to, the detachable needle already connected to the barrel in the syringe assembly. In certain embodiments, the shield apparatus may comprise an empty chamber. When the user is done using the original detachable needle, he may insert it into the empty chamber, as described above, and release the needle from its connection to the syringe. This process may continue as many times as there are fresh needles in the kit. After all of the needles have been used, the shield apparatus or any part of the kit, or the kit itself, can then be discarded safely and conveniently, the user having avoided the risk of accidental injection with a used needle.

An advantage available in such embodiments is that while smaller gauge needles generally cause less discomfort for patients, they are limited in their capacity for drawing up large amounts of liquid quickly. Thus, in these embodiments, the user can draw up the drug faster using a larger gauge needle (for example, an 18 gauge needle), but then interchange that larger gauge needle for a smaller-gauge needle when preparing to inject the drug into the patient. This will save time and energy, and minimize the patient's physical and psychological discomfort in waiting for needles to be prepared in between injections.

For example, a patient may have a desire to complete several Botox® injections or insulin injections quickly, or a patient may be suffering pain or discomfort due to muscle spasms or a related medical condition necessitating a series of injections rapidly administered (for example, a series of injections to treat allergies or cerebral palsy). In such situations, a patient may be impatient, agitated or emotional (this is especially true if the patient is a child), and anything that will streamline the process of injecting, reloading and re-injecting is likely to be desirable from the standpoint of patient comfort. In particular, embodiments of the present invention provide an easy way for a user (such as the medical staff or the patient himself) to go through the entire process of unwrapping a syringe from its packaging, loading the drug (for embodiments wherein the drug is not pre-loaded into the syringe), preparing the appropriate needle for injection, injecting the drug, detaching the needle, discarding the used needle and reloading a fresh needle as necessary, all with minimal effort.

In certain embodiments of the present invention, the shield apparatuses in the kits of the invention are configured such that they capable of standing upright without assistance or intervention; in various embodiments, all of the above steps can be made using a single hand, thus decreasing the chances of accidental puncturing with a used needle and maximizing the user's ability to accomplish the desired results without additional help.

The kits of the present invention can comprise any configuration of syringes assemblies, barrels, needle assemblies (of varying sizes, gauges and types), shield apparatuses and chambers, depending on the needs of the patient or medical staff. In some embodiments, the chambers may be configured so that they are each capable of holding more than one needle.

Exemplary shield assemblies in accordance with the present invention can be seen, for example, in **Figs. 2** and **3** which depict a double-ended shield assembly that may be capable of standing on one end. In various embodiments, the kit may comprise a syringe already inserted into one end of the double-ended shield assembly. A user may pull the syringe out of the end of the shield assembly. In various embodiments, the syringe may either be attached to a higher gauge needle, which the user may then insert into a bottle of drug sought to be injected into the patient's body; or alternatively, the syringe may already be pre-loaded with the drug and may be attached to a needle that is intended for injection into the patient. Alternatively, the syringe may be attached to a needle that is intended for injection into the patient but still not pre-loaded with drug. In accordance with certain embodiments of the invention, the two chambers need not be the same size, and one chamber may be suitable for holding a larger gauge needle than the other chamber.

In other embodiments, a kit of the present invention may comprise a shield apparatus that has only a single chamber, as depicted in **Fig. 1**. A kit of the present invention may comprise multiple shield apparatuses of varying configurations, sizes and needle-holding capabilities, and a user may be able to customize the array of shield apparatuses, based on his unique needs.

In various embodiments, the syringes of the present invention may include handles that are configured for ease of use - for example, a handle having a loop that can easily be grasped using a single hand, or a handle comprising a soft or pliant material that provides a comfortable grip for the user and yields to the pressure of a user's hand.

The syringe barrels in the syringe assemblies of the present invention may have fluid volume capacities, in various embodiments, of about 2 to about 5 mL, about 3 mL, about 2 mL, about 1 mL, about 0.5 mL or about 0.3 mL. It has been found that the assemblies of the present invention provide unexpected benefits when used with very small volume barrels and very small (higher gauge) needles.

The kits, syringe assemblies and other components of the kits of the present invention may be made of any materials that are useful for medical devices, including those that are inert, stable, and can be sterilized. Preferably they will not cause undue discomfort or allergic reactions in users or patients. Examples of useful materials are glass, polymeric materials such as plastic (including but not limited to materials comprising polypropylene, polyethylene, polystyrene, polyethylene terephthalate, or low density or high density forms of any of the foregoing), natural or synthetic rubbers, fiberglass, metal and the like.

All embodiments described herein are illustrative and in no way limit the scope of the invention, and the invention may be embodied in other forms not explicitly described here, without departing from the spirit thereof.

## Claims

1. A kit comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof; and a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space; and
(b) a separate needle assembly that is capable of being detachably fixed to the distal end of the cylindrical barrel to form a syringe assembly, the needle assembly comprising a hub and a needle fixed on the hub, wherein the needle comprises a hollow lumen therethrough, the hollow lumen communicating with the interior space of the cylindrical barrel.

2. The kit of claim 1, further comprising:
(c) a shield apparatus for storing the needle assembly, wherein the shield apparatus comprises a housing having an exterior wall and an interior wall defining an interior chamber that encloses the needle point.

3. The kit of claim 2, wherein the shield apparatus is capable of freely standing on a horizontal plane, such that when the shield apparatus stores a syringe assembly, the needle point is facing substantially vertically downward.

4. The kit of claim 2, comprising two or more needle assemblies that are all capable of being detachably fixed to the distal end of the cylindrical barrel, wherein one or more of the needle assemblies is held within the chamber.

5. The kit of claim 1, wherein the syringe assembly further comprises a bevel indicator on the needle assembly.

6. The kit of claim 1, comprising one syringe assembly and two or more needle assemblies.

7. The kit of claim 6, comprising one syringe assembly and two or more needle assemblies, each including a needle of a different gauge.

8. The kit of claim 7, comprising a needle assembly including a needle having a gauge of 25 or lower.

9. The kit of claim 7, comprising a needle assembly including a needle having a gauge of higher than 25.

10. The kit of claim 1, wherein the needle assembly includes a marking that indicates one or more of the following: the size of the needle attached to the needle assembly, the status of the needle assembly or the direction or configuration of the needle point.

11. A kit comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof; and a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space;
(b) a first needle assembly detachably fixed to the distal end of the cylindrical barrel, the needle assembly comprising a hub and a needle fixed on the hub, wherein the needle comprises a hollow lumen therethrough, the hollow lumen communicating with the interior space of the cylindrical barrel; and
(c) a shield apparatus comprising a housing having an exterior wall and an interior wall defining an interior chamber, the shield apparatus holding a second needle assembly in its interior chamber.

12. A method of forming a syringe assembly, the method comprising the steps of:
(a) obtaining a cylindrical barrel having a proximal end and a distal end, the cylindrical barrel defining an interior space, the interior space including a distal end and a roof on the distal end, the interior space configured to receive a plunger capable of sliding relative to the cylindrical barrel;
(b) obtaining a syringe assembly, the syringe assembly comprising a hub fixed to a needle, the needle having a needle point; and
(c) detachably connecting the distal end of the cylindrical barrel onto the syringe assembly; wherein the detachable connection is achieved with a mechanism chosen from: a threading connection, a snap connection and a binary connection.

13. The method of claim 12, wherein the syringe assembly is held within a shield apparatus in a position such that the needle point is facing substantially vertically downward.

14. The method of claim 12, wherein the cylindrical barrel and the syringe assembly are separately contained within a single kit.
